# EUROPEAN PATENT SPECIFICATION

(11) **EP 2 387 325 B1**
(45) Date of publication and mention of the grant of the patent: **17.08.2016**
(21) Application number: 10700915.1
(22) Date of filing: 14.01.2010
(51) Int. Cl.: A23K 20/121, A23K 50/40, A61P 37/04

(54) **METHODS FOR IMPROVING HEPATIC AND IMMUNE FUNCTION IN AN ANIMAL**
VERFAHREN FÜR DIE VERBESSERUNG DER LEBER- UND IMMUNFUNKTION IN EINEM TIER
PROCÉDÉS D'AMÉLIORATION DE LA FONCTION HÉPATIQUE ET IMMUNITAIRE CHEZ UN ANIMAL

(30) Priority: 14.01.2009 US 353351
(43) Date of publication of application: 23.11.2011
(73) Proprietor: Hill's Pet Nutrition, Inc., Topeka, KS 66603 (US)
(72) Inventor: ZICKER, Steven, C., Lawrence, KS 66049 (US); PAETAU-ROBINSON, Inke, Auburn, KS 66402 (US)
(74) Representative: Daniels, Jeffrey Nicholas
(86) International application number: PCT/US2010/020993
(87) International publication number: WO 2010/083277

(56) References cited:
- EP-A1- 1 529 529
- WO-A1-2010/077903
- WO-A2-2006/058248
- WO-A2-2006/058278
- US-A1- 2005 123 628
- DATABASE WPI Week 200710 Thomson Scientific, London, GB; AN 2007-098516 XP002581224 & KR 2006 072 603 A (HAN SEO PHARM CO LTD) 28 June 2006 (2006-06-28)
- DATABASE WPI Week 200780 Thomson Scientific, London, GB; AN 2007-868163 XP002581225 & KR 2007 025 426 A (BUKWANG PHARM CO LTD) 8 March 2007 (2007-03-08)
- DATABASE WPI Week 200882 Thomson Scientific, London, GB; AN 2008-O13218 XP002608402 & KR 100 811 110 B1 (DPI SOLUTIONS INC) 6 March 2008 (2008-03-06)
- OHMORI H ET AL: "AUGMENTATION OF THE ANTIBODY RESPONSE BY LIPOIC ACID IN MICE II. RESTORATION OF THE ANTIBODY RESPONSE IN IMMUNOSUPPRESSED MICE" JAPANESE JOURNAL OF PHARMACOLOGY, THE JAPANESE PHARMACOLOGICAL SOCIETY, KYOTO, JP LNKD- DOI:10.1254/JJP.42.275, vol. 42, no. 2, 1 January 1986 (1986-01-01), pages 275-280, XP001008328 ISSN: 0021-5198
- CUI J ET AL: "Lipoic acid attenuates high fat diet-induced chronic oxidative stress and immunosuppression in mice jejunum: A microarray analysis" CELLULAR IMMUNOLOGY, ACADEMIC PRESS, SAN DIEGO, CA, US LNKD- DOI:10.1016/J.CELLIMM.2009.08.001, vol. 260, no. 1, 1 January 2009 (2009-01-01), pages 44-50, XP026733967 ISSN: 0008-8749 [retrieved on 2009-08-11]

## Description

### FIELD OF THE INVENTION

The invention encompasses compositions for use in improving immune function in felines. The description further provides compositions and methods for improving hepatic function in felines.

### BACKGROUND OF THE INVENTION

The liver is a vital organ and has an important role in most every bodily function of a mammal. In one role, the liver acts as a filtration system to protect other organs from the effects of toxin buildup. Toxins absorbed from the digestive system are removed from the blood by the liver before they can affect the rest of the body. The capacity of a xenobiotic such as a drug, therapeutic agent, or chemical to produce injury to a liver is known as hepatotoxicity. The xenobiotic is a pharmacologically or toxicologically active substance not indigenously produced and therefore foreign to an organism. Many industrial compounds, drugs and other therapeutic agents are well established as injurious to a liver. As mammals age, their capacity for the filtration and clearance of xenobiotics by the liver decreases. It is well known that as mammals age, especially companion animals, they encounter health problems that require drugs and other therapeutic agents. Since liver filtration and clearance decreases in such an aged animal, administration of such drugs and therapeutic agents to improve the health of the animal may have hepatotoxic effects.

R-α-Lipoic acid (CAS number 1200-22-2, also known as thioctic acid and 1,2-dithiolane-3-pentanoic acid) naturally occurs in plant and animal tissues, where it is covalently bound to an ε-amino group of lysine residues. Lipoic acid is commercially available and is produced by companies such as BASF and Cognis. Lipoic acid is commercially available as an essentially pure R-α lipoic acid or as a racemic mixture of lipoic acid isomers. In plant lipoic acid is most abundant in spinach and potatoes while in animal tissues, lipoic acid is most abundant in the kidney and the beart. R-α-lipoic acid was first discovered in 1937 (See Snell et al., Journal Bact. 33; 207, 1937) and was not isolated and characterized until 195 (See Reed et al, Science 114:94-4, 1951). R-α-lipoic acid may be synthesized and such methods are well known in the art. (See U.S. Patent No. 2,890,716 to Reed issued April 18, 1961). R-α-lipoic acid has been classified as an antioxidant and has been used in high dosages as a treatment for Tye II diabetes. Studies have shown that mixtures of carnitine and lipoic acid may enhance metabolism and alleviate oxidative stress. (See U.S. Patent No. 5,916,912 to Ames et al, issued June 29,1999 and U.S. Patent No. 6,365,622 to Cavayzo issued April 2, 2002). In addition, it has been shown that a companion animal diet comprising lipoic acid among other ingredients appears to inhibit the deterioration of the mental capacity of an aged companion animal. (See U.S. Patent Application Publication Nos. 2002/0076469. 2002/0052402, 2002/0076470, 2000/115710, and 2002/0119182).

Studies have shown that mitochondrial oxidation plays a role in the metabolism of lipoic acid. Although the metabolism in humans mainly resembles that observed in mice and rats, the formation of oxidized structures related to tetranorlipoic acid found in canines appears to have no equivalent in humans. In addition, 3-ketolipoic acid, an intermediate in the mitochondrial oxidation of lipoic acid has been reported in plasma samples from rats and humans but has not been found in plasma from canines. (See Schupke, H. et al. Drug Metabolism and Disposition, 29 (6) 855-862. 2001). It appears that the metabolic pathway of α-lipoic acid is different in canines as compared to humans.

Mercapturic acids are sulfur derivatives of N-acetyl-cysteine, which is synthesized from glutathione (GSH). It is generally accepted that most compounds are metabolized to mercapturic acids first undergo conjugation with GSH catalyzed by an enzyme called glutathione S -transferase, found in the soluble or supernatant liver refractions. The mercapturic acid pathway appears to have evolved as a protective mechanism against xenobiotic induced hepatotoxicity or carcinogenicity, serving to detoxify a large number of noxious substances that are inhaled, ingested or normally produced metabolically every day. Lipoic acid not only up regulates the glutathione but also up regulates the enzyme, glutathione S-transferase that conjugates glutathione in the liver. Bromosulfophthalein (CAS number 71-67-0 also known as BSP and sulfobromophthalein) is an organic dye that, when injected into the circulation, is removed by the liver at a rate that reflects the liver's ability to extract and metabolize a number of organic compounds. See S. M. Rosenthal, E.C. White, J. Pharmacol. 24, 265 (1924) W. Hacki et al., J. Lab. Clin. Med. 88, 1019 (1976). BSP is cleared from the liver in three steps. First, BSP is transferred from albumin through the plasma to the liver. This step is dependent on plasma protein concentration and other ligands that bind to plasma proteins. Secondly, BSP is complexed in the liver by a ligand and z protein. Finally, BSP is conjugated by glutathione via glutathione S-transferase enzyme and eliminated into the bile duct and this is the rate-limiting step. Thus BSP is an example of a xenobiotic that, when measured in the blood after injection, provides information on the functional capabilities of the liver.

WO2006/058248 describes methods for increasing the immune response in an animal.

XP002608402 (Database WP1 Week 200882; Thompson Scientific, London, 2008-013218; DPI Solutions, Inc.) describes a feed additive comprising a water dispersible lipoic acid capsule composition.

EP1529529 describes a new medicament for treating thiol-disulfide status disorders in diabetes mellitus comprising a glutathione metabolism effector and α-lipoic acid.

Ohmori H. et al. (Japan. J. Pharmacol. 42, 275-280 (1986)), describes an augmentation of the antibody response by lipoic acid in mice and restoration of the antibody response in immunosuppressed mice.

Cui J. et al (Cellular Immunology, 260 (2009) 44-50) describes the use of lipoic acid in attenuating high fat diet-induced chronic oxidative stress and immunosuppression in mice jejunum.

### SUMMARY OF THE INVENTION

The present invention provides a composition for use in improving immune function in a feline in need thereof, wherein the improvement in immune function comprises increasing lymphocyte proliferation, which composition comprises an effective amount of lipoic acid or a salt thereof, wherein said effective amount is effective in improving immune function in a feline, and wherein the lipoic acid or salt thereof is part of the feline's daily diet.

The present invention further provides a composition comprising an effective amount of lipoic acid or a salt thereof to improve immune function in a feline, wherein said effective amount of lipoic acid is 650 ppm, and wherein the improvement in immune function comprises increasing lymphocyte proliferation, and wherein the composition is a food composition.

Further areas of applicability of the present invention will become apparent from the detailed description provided hereinafter.

### BRIEF DESCRIPTION OF THE DRAWINGS

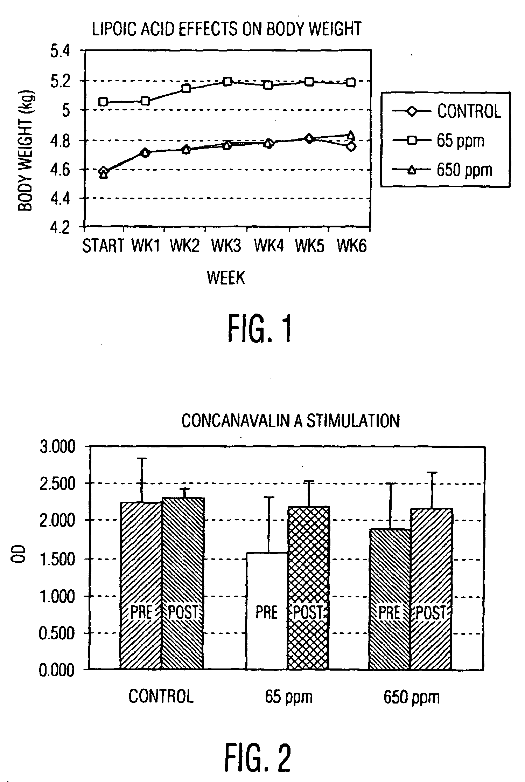

Figure 1 is a graphical representation illustrating that the inclusion of lipoic acid into foods at 65 ppm and 650 ppm for 6 weeks had no adverse effects on bodyweight for cats.
Figure 2 illustrates the effect of inclusion of 65 ppm and 650 ppm in diets and time on Concanavalin A stimulation. There was no significant difference between the groups at the beginning of the study or at the end. However, the cats on the 65 ppm lipoic acid inclusion had a significant increase in Concanavalin A-activated lymphocyte proliferation compared to baseline. Cats on the 650 ppm also displayed an increase.

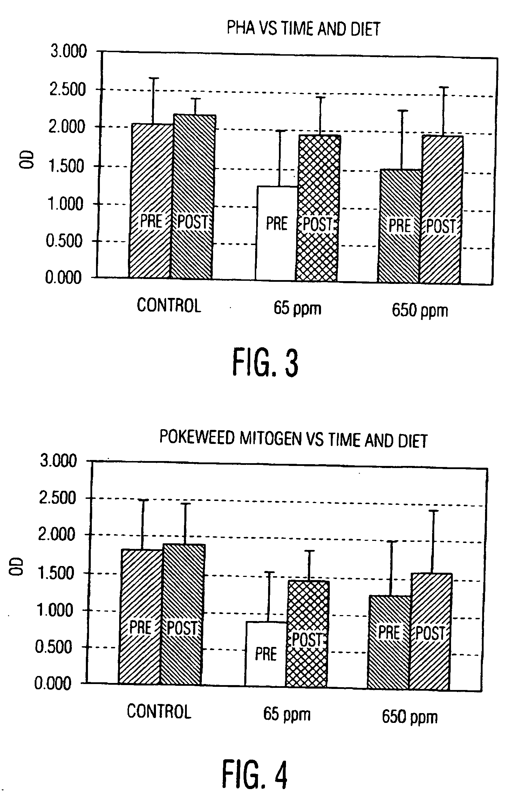

Figure 3 illustrates that phytohaemagglutinin (PHA) stimulation showed no significant difference between groups at beginning or end. The illustrative group administered 65 ppm lipoic acid had a significant increase in lymphocyte proliferation between baseline and 6 weeks of intervention.
Figure 4 illustrates Pokeweed Mitogen stimulation was different between groups (ANOVA P<0.05) at the beginning but not the end of the study. The group with the lowest starting mean was the 65 ppm group. Subsequently, lymphocyte proliferation in the group administered 65 ppm lipoic acid was significantly increased compared to baseline after 6 weeks time.

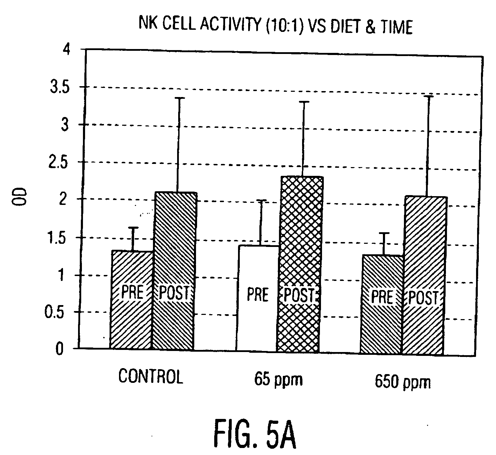

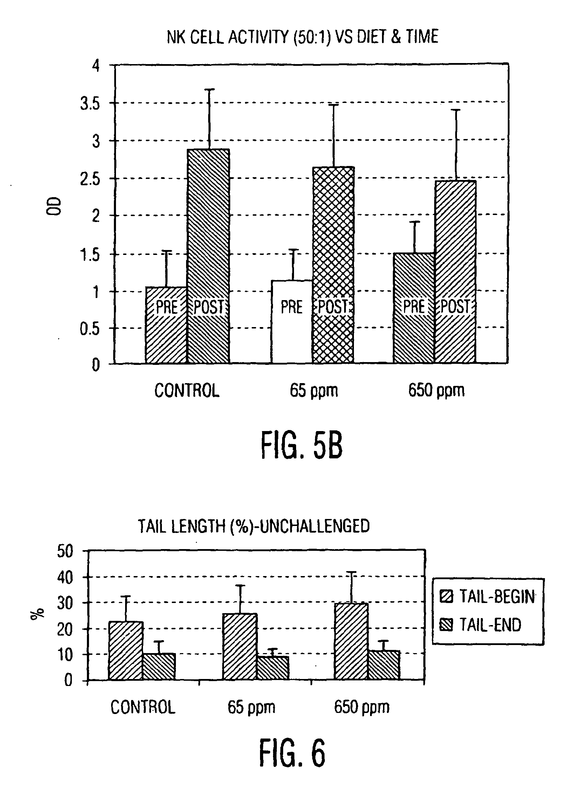

Figure 5a and 5b illustrate natural killer cell activity. Based on the illustrative studies, no significant changes were detected from baseline to end of study for the 10:1 stimulation rate; however, all p values were less than 0.1. All changes between baseline and end of study were significant for all groups at the 50:1 stimulation rate.
Figure 6 illustrates graphs for representative Comet assays. Two illustrative assays were performed: (1) inherent DNA damage and (2) hydrogen peroxide challenged damage. Analysis of the data showed that all head DNA for all diets increased significantly (P<0.05) over the duration of the study for both. In addition, all tail DNA tail length and Olive tail moments decreased during the duration of the study for both control and hydrogen peroxide challenged comet tests. However, there were no significant differences between groups at either the beginning or end for any of the comet measures. In addition, ANOVA analysis of the change over time (difference pre-post) showed no significant difference via t-test for each comet variable under control and hydrogen peroxide challenge conditions.

The Figures are intended to exemplify the general characteristics of the invention for the purposes of the description of such embodiments herein. The Figure may not precisely reflect the characteristics of any given embodiment and is not necessarily intended to define or limit specific embodiments within the scope of this invention.

### DETAILED DESCRIPTION OF THE INVENTION

### Definitions

The term "animal" as used in the description means any animal susceptible to or suffering from impaired liver function and in need of improved liver clearance of xenobiotic substances or an animal that could benefit from improved liver clearance of xenobiotic substances. An animal is "susceptible to" a disease or condition if the animal exhibits symptoms that indicate that the animal is likely to develop the condition or disease. An animal is "suffering from" a disease or condition if the animal exhibits symptoms that are indicative that the animal has developed the condition or disease.

As used herein, the terms "lipoic acid or a salt thereof" includes, but is not limited to, for example, alpha-lipoic acid, a racemic mixture of lipoic acids, a lipoate salt, ester, amide or derivative thereof, for example as described in U.S. patent number 5,621,117. In various embodiments, the lipoic acid can be administered in a composition comprising a wet or dry food composition, which may be in the form of a moist food, dry food, supplement or treat. The lipoic acid may be incorporated therein or on the surface of any food composition, such as, by spraying or precipitation thereon or may be added to the diet by way of snack, supplement, treat or in the liquid portion of the diet such as water or another fluid. The lipoic acid may be administered as a powder, solid or as a liquid including a gel. An important aspect is that the animal be provided an effective amount of the lipoic acid to provide a positive effect. Typically, the source of lipoic acid or a salt thereof is present in the composition in an amount of up to an amount which remains non-toxic to the animal.

The phrase "salt thereof," as used herein includes but is not limited to salts of lipoic acid used in the pet food compositions. Lipoic acid is acidic in nature and therefore is capable of forming base salts with various cations. Examples of such salts include alkali metal or alkaline earth metal salts and, particularly, calcium, magnesium, sodium lithium, zinc, potassium, and iron salts.

The term "older animal" means any animal susceptible to or suffering from impaired liver function and in need of improved liver clearance of xenobiotic substances or an animal that could benefit from improved liver clearance of xenobiotic substances because of age.

### General Description

The invention generally encompasses compositions comprising an effective amount of lipoic acid or a salt thereof, wherein said effective amount is effective in improving immune function in an animal.

The description further generally provides compositions comprising an effective amount of lipoic acid or a salt thereof, wherein said effective amount is effective in improving hepatic function in an animal.

The invention specifically provides a composition for use in improving immune function in a feline in need thereof, wherein the improvement in immune function comprises increasing lymphocyte proliferation, which composition comprises an effective amount of lipoic acid or a salt thereof, wherein said effective amount is effective in improving immune function in a feline, and wherein the lipoic acid or salt thereof is part of the feline's daily diet.

In an embodiment, the effective amount is from 50 ppm to 1200 ppm of lipoic acid or a salt thereof.

In another embodiment, the effective amount is from 65 ppm to 650 ppm of lipoic acid or a salt thereof.

In another embodiment, the daily diet comprises lipoic acid or salt thereof in an amount of greater than 25 ppm on a dry weight basis.

In another embodiment, the composition is a food composition.

In another embodiment, the food composition is a dry food composition.

In another embodiment, the food composition is extruded.

In another embodiment, the food composition is canned.

In another aspect, the description provides methods for improving hepatic or immune function in an animal comprising feeding an effective amount of lipoic acid or a salt thereof to the animal, wherein said effective amount is effective in improving hepatic or immune function.

In certain aspects, the methods are effective in improving hepatic function in an animal.

In other aspects, the methods are effective in improving immune function in an animal.

In another aspect, the effective amount is from 25 ppm to 2600 ppm of lipoic acid or a salt thereof.

In another aspect, the effective amount is from 50 ppm to 1200 ppm of lipoic acid or a salt thereof.

In another aspect, the effective amount is from 65 ppm to 650 ppm of lipoic acid or a salt thereof.

In another aspect, the animal is a companion animal.

In another aspect, the companion animal is a feline.

In another aspect, the lipoic acid is part of the animal's daily diet.

In another aspect, the daily diet comprises lipoic acid in an amount of greater than 50 ppm on a dry weight basis.

In another aspect, the lipoic acid is fed to the animal in a food composition suitable for consumption by the animal.

In another aspect, the animal is an older animal.

The present invention additionally encompasses a composition comprising an effective amount of lipoic acid or a salt thereof to improve immune function in a feline, wherein said effective amount of lipoic acid is 650 ppm, and wherein the improvement in immune function comprises increasing lymphocyte proliferation, and wherein the composition is a food composition.

In certain embodiments, the composition is a dry food composition.

In another embodiment, the composition is extruded or canned.

### Compositions of the Invention

The quantity of alpha-lipoic acid in the compositions provided herein can vary from at least about 25 ppm, about 50 ppm, about 100 ppm, about 200 ppm, about 300 ppm, about 500 ppm, about 700 ppm, about 900 ppm, about 1100 ppm, about 1200 ppm, about 1400 ppm, about 1600 ppm, about 1800 ppm, about 2000 ppm, about 2200 ppm, about 2400 ppm, or about 2600 ppm.

In various embodiments, the range of lipoic acid that can be administered to cats is 25 ppm to 2600 ppm. In certain illustrative embodiments, quantities can vary 65 ppm to an amount which remains nontoxic to the pet. In other embodiments, a range is 50 ppm to 1200 ppm. In other embodiments, a range is 65 ppm to 650 ppm.

In various embodiments, a food composition comprising lipoic acid provides a substantially nutritionally complete diet for the intended recipient animal. A "nutritionally complete diet" is a diet that includes sufficient nutrients for maintenance of normal health of a healthy animal on the diet.

The lipoic acid or salt thereof is present at a concentration that is not deleterious to the intended animal's health. Thus, for example, the lipoic acid or salt thereof is present at a concentration that does not cause undesirable or toxic effects.

The composition can be a liquid or a solid food. When the composition is a liquid, the lipoic acid or salt thereof can be admixed with other components. Where the composition is solid, the lipoic acid may be coated on the composition, incorporated into the composition, or both.

In various embodiments, the lipoic acid or salt thereof may be added to the animal's food. In certain embodiments, the lipoic acid or salt thereof may be added to the animal's food by a compounder or manufacturer at a site or by an animal's caregiver prior to feeding the animal. In other embodiments, the lipoic acid or salt thereof may be added during the processing of an animal's food, such as during and/or after mixing of other components of the composition that is then packaged and made available to consumers. Such processing may include extrusion, canning, baking, and the like or any other method or process of producing pet foods that is known in the art. In other embodiments, the lipoic acid or salt thereof may be contributed by a natural source like an animal or plant component or the lipoic acid or salt thereof may be contributed try a synthetically derived source, or the acid or salt thereof may be contributed by a mixture of natural and synthetic sources.

The compositions in addition to lipoic acid or a salt, thereof include at least one component suitable for consumption by a companion animal including, but not limited to, fats, carbohydrates, proteins, fibers, nutritional balancing agents such as vitamins, minerals, and trace elements, and mixtures thereof. One of ordinary skill in the art can select the amount and type of food ingredients for a typical food based upon the dietary requirements of the animal, for example, the animal's species, age, size, weight, health, and function.

The food ingredient part of the food composition can include up to about 100% of any particular food ingredient or can include a mixture of food ingredients in various proportions. In certain embodiments, the food composition includes a combination of food ingredients in amounts of 0 wt. % to 50 wt. % fat, 0 wt. % to 75 wt. % carbohydrate, 0 wt. % to 95 wt. % protein, 0 wt. % to 40 wt. % dietary fiber, and 0 wt. % to 15 wt. % of one or more nutritional balancing agents.

In certain embodiments, the fat and carbohydrate food ingredient is obtained from a variety of sources such as animal fat, fish oil. vegetable oil, meat, meat by-products, grains, other animal or plant sources, and mixtures thereof. Grains include wheat, corn, barley, and rice.

In certain embodiment the protein food ingredients is obtained from a variety sources such as plants, animals, or both. Animal protein includes meat, meat by-products, dairy, and eggs. Meats include the flesh from poultry, fish, and animals such as cattle, swine, sleep, goats, and the life, meat by-products include lungs, kidney, brain, livers, stomachs, and intestines. The protein food ingredients may also be free amino acids and/or peptides. Preferably, the protein food ingredient includes meat, a meat by-product, dairy products, or eggs.

In certain embodiments, the fiber food ingredient is obtained from a variety of sources such as vegetable fiber sources, for example, cellulose, beet pulp, peanut hulls, and soy fiber.

In certain embodiments, the nutritional balancing agents are obtained from a variety of sources know to skilled artisans, for example, vitamin and mineral supplements and food ingredients. Vitamins and minerals can be included in amounts required to avoid deficiency and maintain health. These amounts are readily available in the art. The National Research Council (NRC) provides recommenced amounts of such nutrients for farm animals. See, e.g., Nutrient Requirements of Swine (10th Rev. Ed., Nat'l Academy Press, Wash, D.C., 1998), Nutrient Requirements of Poultry (9th Rev. Ed., Nat'l Academy Press, Wash. D.C., 1994), Nutrient Requirement of Horses (5the Rev. Ed., Nat'l Academy Press, Wash. D.C., 1989). The American Feed Control Officials (AAFCO) provides recommended amounts of such nutrients for dogs and cats. See America feed Control Officials, Inc., Official publication, pp. 129-137 (2004). Vitamins generally useful as food additives include vitamin A, vitamin B1, vitamin B2, vitamin B6, vitamin B12, vitamin D, biotin, vitamin K, folic acid, inositol, niacin, and pantothenic acid. Minerals and trace elements useful as food additives include calcium, phosphorus, sodium, potassium, magnesium, copper, zinc, chloride, iron, selenium, iodine, and iron.

### Reparation of the Composition of the Invention

The compositions of the invention may be prepared in a canned or wet form using conventional food preparation processes known to skilled artisans. Typically, ground animal proteinaceous tissues are mixed with the other ingredients such as fish oils, cereal grains, balancing ingredients, special purpose additives (e.g., vitamin and mineral mixtures, inorganic salts, cellulose and beet pulp, bulking agents, and the like) and water in amounts sufficient for processing. These ingredients are mixed in a vessel suitable for heating while blending the components. Heating of the mixture is effected using any suitable manner, for example, direct steam injection or using a vessel fitted with a heal exchanger. Following the additionally least ingredient, the mixture is heated to a temperature of about 10°C (50°F) to about 100°C (212 °F). Temperatures outside this range are acceptable but may be commercially impractical without use of other processing aids. When heated to the appropriate temperature, the material will typically be in the form of a thick liquid. The thick liquid is filled into cans. A lid in applied, and the container is hermetically sealed. The sealed can is then placed into conventional equipment designed to sterilize the contents. Sterilization is usually accomplished by heating to temperatures of greater than about 110°C (230 °F) for an appropriate time depending on the temperature used, the composition, and similar factors. The compositions of the present invention can be added to the food compositions before, during, or after preparation.

Food compositions may be prepared in a dry form using conventional processes knows to skilled artisans. Typically, dry ingredients such as animal protein, plant protein, grains, and the like are ground and mixed together. Moist or liquid ingredients, including fats, oils, animal protein, water, and the like are then added to and mixed with the dry mix. The mixture is then processed into kibble or similar dry pieces. Kibble is often formed using an extrusion process in which the mixture of dry and wet ingredients is subjected to mechanical work at a high pressure and temperature and forced through small openings and cut off into kibble by a rotating knife. The wet kibble is then dried and optionally coated with one or more topical coatings such as flavors, fats, oils, powders, and the like. Kibble also can be made from the dough using a baking process, rather than extrusion, wherein the dough is placed into a mold before dry-heat processing. The food compositions can be in the form of a treat using an extrusion or baking process similar to those described above for dry food or a toy such as those disclosed in U.S. Patent Nos. 5,339,771 and 5,419,283. The compositions of the present invention can be added to the food compositions before, during, or after preparation.

### Methods of the Description

The description also encompasses methods for improving hepatic function in animals. The methods include feeding an amount of lipoic acid or a salt thereof effective to improve hepatic function to an animal in need thereof. Generally, the lipoic acid is feed to the animal in amounts of 25 ppm to 2600 ppm. In certain illustrative embodiments, quantities can vary from 25 ppm to 2600 ppm or to an amount which remains nontoxic to the pet. In other embodiments, a range is 50 ppm to 1200 ppm. In other embodiments, a range is 65 ppm to 650 ppm.

The description also encompasses methods for improving immune function in animals. The methods include feeding an amount of lipoic acid or a salt thereof in an amount effective to improve immune function to the animal in need thereof. Generally, the lipoic acid is feed to the animal in amounts of 25 ppm to 2600 ppm. In certain illustrative embodiments, quantities can vary from 25 ppm to 2600 ppm or to an amount which remains nontoxic to the pet. In other embodiments, a range is 50 ppm to 1200 ppm. In other embodiments, a range is 65 ppm to 650 ppm.

The methods of the description include feeding an animal, for example, a companion animal such as a feline, a composition or diet containing lipoic acid or a salt thereof to improve hepatic function or immune function, particularly when these functions may be impaired by age, and to improve the overall health of the animal. The amount of lipoic acid given to the animal is a non-toxic amount. The lipoic acid may be either provided to the animal as a supplement or contained in a composition, including a diet, fed to the animal. Such a supplement may be in the form of a pill or capsule, a treat or a biscuit, or any other edible form. By "diet," it is meant the food or drink regularly consumed by the animal. A diet may include supplements consumed by the animal. A diet is considered to have essentially enough nutrients to be life sustaining for the animal. A companion animal diet can be any suitable pet food formula, which also provides adequate nutrition for the animal. For example, a typical feline diet for use in the present invention may contain from 8 to 50% fat, 16 to 50% by weight protein and 3 to 15% total dietary fiber. In another example, a typical feline diet may contain from 8 to 50% by weight fat and from 30 to 60% by weight protein. However, no specific ratios or percentages of these or other nutrients are required. A nutrient is any food constituent that helps support life. Nutrients important to an animal's health are known to skilled artisans, for example, proteins, carbohydrates, fats, fibers, vitamins, and minerals. Water is also vital to an animal's health.

Various aspects of the description include a method for improving hepatic function or immune function in an animal, particularly a companion animal. In such aspects, the method comprises feeding to the animal a composition, for example a diet, comprising lipoic acid or a salt thereof in an amount of at least 25 ppm on a dry matter basis. In still other aspects, the method comprises feeding to the animal a diet comprising lipoic acid in an amount from 65 ppm to 650 ppm on a dry matter basis. As used herein, lipoic acid is in a racemic mixture, but other embodiments may include lipoic acid which is essentially pure R-α lipoic acid or as a lipoate derivative, mixtures of isomers, salts, esters, amides or combinations thereof (For example see US Patent No 5,621,177 to Bethge et al. issued April 15, 1997). In various aspects, the range of lipoic acid that can be administered cats is 25 ppm to 2600 ppm. In certain illustrative aspects, quantities can vary from 65 ppm to 2600 ppm or to an amount which remains nontoxic to the pet. In other aspects, a range is 50 ppm to 1200 ppm. In other embodiments, a range is 65 ppm to 650 ppm.

The present invention specifically provides a composition for use in improving immune function in a feline in need thereof, wherein the improvement in immune function comprises increasing lymphocyte proliferation, which composition comprises an effective amount of lipoic acid or a salt thereof, wherein said effective amount is effective in improving immune function in a feline, and wherein the lipoic acid or salt thereof is part of the feline's daily diet.

In various aspects of the description, there is provided a composition or diet comprising at least 25 ppm to 2600 ppm of lipoic acid or a salt thereof. In some aspects, the lipoic acid or salt thereof is added to the companion animal's food. In such aspects, the lipoic acid or salt thereof may be added during the processing of the companion animal food that is then packaged and made available to consumers. Such processes may include extrusion, canning, baking and the like or any other method or process of producing pet foods that is known in the art. In such processes, the lipoic acid may be contributed by a natural source like an animal or plant component, such as kidney or spinach or the lipoic acid may be contributed by a synthetically derived source, or the lipoic acid may be contributed by a mixture of natural and synthetic sources. In other aspects, lipoic acid may be in a capsule form to be fed to the companion animal. In still other aspects, the lipoic acid or salt thereof may be in a powder or in a crystalline, which may be added to the animal's food or fed directly to the animal. In various aspects, the companion animal diet comprises lipoic acid or salt thereof and other needed nutritional components. In various aspects, the companion animal is a dog and in other embodiments, the companion animal is a cat.

In a further aspect, the present description provides for a use of lipoic acid or salt thereof to prepare a medicament. In another, the description provides for the use of lipoic acid to prepare a medicament for maintaining and/or improving animal health, e.g., improving hepatic function or immune function in an animal by feeding an amount of lipoic acid or a salt thereof to the animal. Generally, medicaments are prepared by admixing a compound or composition with excipients, buffers, binders, plasticizers, colorants, diluents, compressing agents, lubricants, flavorants, moistening agents, and other ingredients known to skilled artisans to be useful for producing medicaments and formulating medicaments that are suitable for administration to an animal.

In a further aspect, the description provides kits suitable for feeding lipoic acid or salt thereof to an animal. The kits comprise in separate containers in a single package or in separate containers in a virtual package, as appropriate, lipoic acid and at least one of (1) one or more ingredients suitable for consumption by an animal, (2) instructions for how to combine the lipoic acid and other kit components to improve liver clearance of xenobiotic substances, particularly to produce a composition useful for improving liver clearance of xenobiotic substances, and (3) instructions for how to use the lipoic acid and other components of the present invention, particularly for the benefit of the animal. When the kit comprises a virtual package, the kit is limited to instructions in a virtual environment in combination with one or more physical kit components. The kit contains the lipoic acid and other components in amounts sufficient to improve liver clearance of xenobiotic substances. Typically, the lipoic acid and the other suitable kit components are admixed just prior to consumption by an animal. In one aspect, the kit contains a packet containing lipoic acid and a container of food for consumption by an animal. The kit may contain additional items such as a device for mixing the lipoic acid and ingredients or a device for containing the admixture, e.g., a food bowl. In another aspect, the lipoic acid is mixed with additional nutritional supplements such as vitamins and minerals that promote good health in an animal.

As used herein and in the appended claims, the singular forms "a," "an," and "the" include plural reference unless the context clearly dictates otherwise. The terms "comprise", "comprises", and "comprising" are to be interpreted inclusively rather than exclusively.

Unless defined otherwise, all technical and scientific terms and any acronyms used herein have the same meanings as commonly understood by one of ordinary skill in the art in the field of the invention. Although any methods and materials similar or equivalent to those described herein can be used in the practice of the present invention, the preferred methods, devices, and materials are described herein.

### EXAMPLES

This invention can be further illustrated by the following examples of preferred embodiments thereof, although it will be understood that these examples are included merely for purposes of illustration and are not intended to limit the scope of the invention unless otherwise specifically indicated.

### Example 1

The study involved three groups of cats: Group 1) cats on a dry control food, Group 2) cats on a dry food fortified with approximately 65 ppm of lipoic acid, and Group 3) cats on a dry food fortified with approximately 650 ppm of lipoic acid on a dry matter basis. All cats were fed control food for a two week baseline period at the end of which time immune function assays were performed. One group of cats was then switched to the 65 ppm test food, one group to the 650 ppm test food and one group remained on control and all were fed for another 6 weeks at which time the baseline tests were performed again.

The administration of lipoic acid to old cats improved lymphocyte proliferation activity, which may improve immune function. Most notably the effect of lipoic acid on improved lymphocyte proliferation in healthy cats at a single level of inclusion (*e.g..* 65 ppm).

As illustrated in Figure 1, the inclusion of lipoic acid into feline pet foods at 65 ppm and 650 ppm for 6 weeks had no adverse effects on bodyweight for cats.

Figure 2 illustrates the effect of inclusion of 65 ppm and 650 ppm in diets and time on Concanavalin A stimulation. There was no significant difference between the groups at the beginning of the study or at the end. However, the cats on the 65 ppm lipoic acid inclusion had a significant increase in Concanavalin A-activated lymphocyte proliferation compared to baseline. Cats on the 650 ppm also displayed an increase. Accordingly, based on the Concanavalin A-activated lymphocyte proliferation, the addition of 65 ppm or 650 ppm lipoic acid to a cat food composition could increase the immune response of cats.

Figure 3 illustrates that phytohaemagglotinin (PHA) stimulation showed no significant difference between groups at beginning or end. The illustrative group administered 65 ppm lipoic acid had a significant increase in lymphocyte proliferation between baseline and 6 weeks of intervention. Accordingly, based on. the PHA-activated lymphocyte proliferation, the addition of 65 ppm or 650 ppm lipoic acid to a cat food composition could increase the immune response of cats

Figure 4 illustrates Pokeweed Mitogen stimulation was different between groups (ANOVA P<0.05) at the beginning but not the end of the study. The group with the lowest starting mean was the 65 ppm group. Subsequently, lymphocyte proliferation in the group administered 65 ppm lipoic acid was significantly increased compared to baseline after 6 weeks time. Accordingly, based on the Pokeweed mitogen-activated lymphocyte proliferation, the addition of 65 ppm or 650 ppm lipoic acid to a cat food composition could increase the immune response of cats

Figure 5a and 5b illustrate natural killer cell activity. Based on the illustrative studies, no significant changes were detected from baseline to end of study for the 10:1 stimulation rate; however, all p values were less than 0.1. All changes between baseline and end of study were significant for all group at the 50:1 stimulation rate.

Figure 6 illustrates graphs for representative Comet assays. Two illustrative assays where performed: (1) inherent DNA damage and (2) hydrogen peroxide challenged damage. Analysis of the data showed that all head DNA for all diets increased significantly (P<0.05) over the duration of the study for both. In addition, all tail DNA tail length and Olive tail moments decreased during the duration of the study for both control and hydrogen peroxide challenged comet tests. However, there were no significant differences between groups at either the beginning or end for any of the comet measures. In addition, ANOVA analysis of the change over time (difference pre-post) showed no significant difference via t-test for each comet variable under control and hydrogen peroxide challenge condition.

In the specification, there have been disclosed typical preferred embodiments of the invention and, although specific terms are employed, they are used in a generic and descriptive sense only and not for purposes of limitation the scope ot the invention being set forth in the folowing claims.

## Claims

1. A composition for use in a method of improving immune function in a feline in need thereof, wherein the improvement in immune function comprises increasing lymphocyte proliferation, which composition comprises an effective amount of lipoic acid or a salt thereof, wherein said effective amount is effective in improving immune function in a feline, and wherein the lipoic acid or salt thereof is part of the feline's daily diet.

2. The composition of claim 1 for the use of claim 1, wherein the effective amount is from 25 ppm to 2600 ppm of lipoic acid or a salt thereof.

3. The composition of any preceding claim for the use of any preceding claim, wherein the effective amount is from 50 ppm to 1200 ppm of lipoic acid or a salt thereof.

4. The composition of any preceding claim for the use of any preceding claim, wherein the effective amount is from 65 ppm to 650 ppm of lipoic acid or a salt thereof.

5. The composition of any preceding claim for the use according to any preceding claim, wherein the daily diet comprises lipoic acid or salt thereof in an amount of greater than 25 ppm on a dry weight basis.

6. The composition of any preceding claim for the use of any preceding claim, wherein the composition is a dry food composition.

7. The composition of any preceding claim for the use of any preceding claim, wherein the feline is an older feline.

8. A composition comprising an effective amount of lipoic acid or a salt thereof to improve immune function in a feline, wherein said effective amount of lipoic acid is 650 ppm, wherein the improvement in immune function comprises increasing lymphocyte proliferation, and wherein the composition is a food composition.

9. The composition of claim 8, wherein the food composition is a dry food composition.

10. The composition of claim 8, wherein the food composition is extruded.

11. The composition of claim 8, wherein the food composition is canned.

## Patentansprüche

1. Zusammensetzung zur Verwendung in einem Verfahren zur Verbesserung der Immunfunktion bei einer Katze mit einem Bedarf daran, wobei die Verbesserung der Immunfunktion die Steigerung der Lymphozytenproliferation umfasst, welche Zusammensetzung eine wirksame Menge Liponsäure oder ein Salz davon enthält, wobei die wirksame Menge bei der Verbesserung der Immunfunktion bei einer Katze wirksam ist, und wobei die Liponsäure oder ein Salz davon einen Teil der täglichen Kost einer Katze bildet.

2. Zusammensetzung nach Anspruch 1 zur Verwendung nach Anspruch 1, wobei die wirksame Menge von 25 ppm bis 2600 ppm Liponsäure oder ein Salz davon ist.

3. Zusammensetzung nach einem vorangehenden Anspruch zur Verwendung gemäß einem vorangehenden Anspruch, wobei die wirksame Menge von 50 ppm bis 1200 ppm Liponsäure oder ein Salz davon ist.

4. Zusammensetzung nach einem vorangehenden Anspruch zur Verwendung gemäß einem vorangehenden Anspruch, wobei die wirksame Menge von 65 ppm bis 650 ppm Liponsäure oder ein Salz davon ist.

5. Zusammensetzung nach einem vorangehenden Anspruch zur Verwendung gemäß einem vorangehenden Anspruch, wobei die tägliche Kost Liponsäure oder ein Salz davon in einer Menge größer als 25 ppm bezogen auf eine Trockengewichtsbasis enthält.

6. Zusammensetzung nach einem vorangehenden Anspruch zur Verwendung gemäß einem vorangehenden Anspruch, wobei die Zusammensetzung eine Trockenfutterzusammensetzung ist.

7. Zusammensetzung nach einem vorangehenden Anspruch zur Verwendung gemäß einem vorangehenden Anspruch, wobei die Katze eine ältere Katze ist.

8. Zusammensetzung, umfassend eine wirksame Menge Liponsäure oder ein Salz davon zur Verbesserung der Immunfunktion bei einer Katze, wobei die wirksme Menge Liponsäure 650 ppm beträgt, wobei die Verbesserung der Immunfunktion eine Steigerung der Lymphozytenproliferation umfasst und wobei die Zusammensetzung eine Futterzusammensetzung ist.

9. Zusammensetzung nach Anspruch 8, wobei die Futterzusammensetzung eine Trockenfutterzusammensetzung ist.

10. Zusammensetzung nach Anspruch 8, wobei die Futterzusammensetzung extrudiert wird.

11. Zusammensetzung nach Anspruch 8, wobei die Futterzusammensetzung eingedost wird.

## Revendications

1. Composition à utiliser dans un procédé d'amélioration de la fonction immunitaire chez un félin qui la nécessite, dans laquelle l'amélioration de la fonction immunitaire comprend l'intensification de la prolifération des lymphocytes, laquelle composition comprend une quantité efficace d'acide lipoïque ou d'un sel de ce dernier, dans laquelle ladite quantité efficace est efficace dans l'amélioration de la fonction immunitaire chez un félin, et dans laquelle l'acide lipoïque ou le sel de ce dernier fait partie du régime alimentaire quotidien du félin.

2. Composition selon la revendication 1, dans laquelle la quantité efficace est comprise entre 25 ppm et 2600 ppm d'acide lipoïque ou d'un sel de ce dernier.

3. Composition selon l'une quelconque des revendications précédentes à utiliser selon l'une quelconque des revendications précédentes, dans laquelle la quantité efficace est comprise entre 50 ppm et 1200 ppm d'acide lipoïque ou d'un sel de ce dernier.

4. Composition selon l'une quelconque des revendications précédentes à utiliser selon l'une quelconque des revendications précédentes, dans laquelle la quantité efficace est comprise entre 65 ppm et 650 ppm d'acide lipoïque ou d'un sel de ce dernier.

5. Composition selon l'une quelconque des revendications précédentes à utiliser selon l'une quelconque des revendications précédentes, dans laquelle le régime alimentaire quotidien comprend de l'acide lipoïque ou du sel de ce dernier en une quantité supérieure à 25 ppm de matière sèche.

6. Composition selon l'une quelconque des revendications précédentes à utiliser selon l'une quelconque des revendications précédentes, dans laquelle la composition est une composition d'aliments déshydratés.

7. Composition selon l'une quelconque des revendications précédentes à utiliser selon l'une quelconque des revendications précédentes, dans laquelle le félin est un félin d'un âge plus avancé.

8. Composition comprenant une quantité efficace d'acide lipoïque ou d'un sel de ce dernier pour l'amélioration de la fonction immunitaire d'un félin, dans laquelle ladite quantité efficace d'acide lipoïque est de 650 ppm, dans laquelle l'amélioration de la fonction immunitaire comprend l'intensification de la prolifération des lymphocytes, et dans laquelle la composition est une composition alimentaire.

9. Composition selon la revendication 8, dans laquelle la composition alimentaire est une composition alimentaire déshydratée.

10. Composition selon la revendication 8, dans laquelle la composition alimentaire est extrudée.

11. Composition selon la revendication 8, dans laquelle la composition alimentaire est mise en conserve.
